# EUROPEAN PATENT SPECIFICATION

(11) **EP 1 061 846 B1**
(45) Date of publication and mention of the grant of the patent: **03.03.2010**
(21) Application number: 99912477.9
(22) Date of filing: 12.03.1999
(51) Int. Cl.: A61M 29/00, A61B 17/12, A61B 17/22, A61F 2/01

(54) **PROTECTIVE DEVICE AGAINST EMBOLIZATION IN CAROTID ANGIOPLASTY**
SCHUTZVORRICHTUNG GEGEN EMBOLISIERUNG BEI KAROTIDANGIOPLASTIE
DISPOSITIF PROTECTEUR CONTRE LES RISQUES D'EMBOLIE DANS L'ANGIOPLASTIE DE LA CAROTIDE

(30) Priority: 13.03.1998 AR P980101146; 13.05.1998 US 78263
(43) Date of publication of application: 27.12.2000
(60) Divisional application: 09005390.1
(73) Proprietor: Gore Enterprise Holdings, Inc., Newark, DE 19714-9206 (US)
(72) Inventor: PARODI, Juan, Carlos, Ciudad de Buenos Aires (AR)
(74) Representative: Shanks, Andrew
(86) International application number: PCT/US1999/005469
(87) International publication number: WO 1999/045835

(56) References cited:
- EP-A- 0 427 429
- US-A- 5 011 488
- US-A- 5 030 227
- US-A- 5 071 407
- US-A- 5 102 415
- US-A- 5 221 261
- US-A- 5 439 446
- US-A- 5 522 882
- US-A- 5 601 591
- US-A- 5 639 274
- US-A- 5 653 689
- US-A- 5 669 927

## Description

### Technical Field

This invention relates to a protective device against embolization in vascular, preferably carotid angioplasty. The device comprises a leading catheter which may be placed in a vascular duct, a tube, which may be inserted through the catheter, and an occluder at the distal end of the tube. The occluder is expandable against the vascular duct to occlude the anterograde blood flow. The occluder has a mouth for drainage of the retrograde blood flow, which allows the retrograde blood flow with any emboli to drain through the internal passageway of the occluder and through the tube to be discharged outside of the patient's body. The invention allows the temporary reversal of the anterograde flow of blood to prevent emboli from reaching the brain and allows for the drainage of emboli to the outside of the patient's body.

A method against embolization, which does not form part of the invention, may comprise the use of the device in conjunction with vacuum suction to allow for the reversal of blood flow to prevent emboli from reaching the brain and to allow the emboli to be drained from the body, cleaned and for the blood to be reinfused into the patient's blood stream.

### Background of the Invention

Although traditionally conventional open surgery has been used in treatments of vascular diseases, such as stenosis of the carotid artery, nowadays endovascular treatments are gaining acceptance. Endovascular treatments are carried out in the lumen of the vascular duct and have the advantage of being less aggressive than the conventional open surgery posing less risk to the patient because it can be performed under limited local anaesthesia.

Where there is stenosis of an artery, such as the common carotid artery, the internal carotid artery or the external carotid artery, the vascular wall of the artery is affected by a pathologic narrowing that prevents the blood stream from flowing normally. A common treatment consists of endovascular angioplasty, where an angioplasty balloon (6) is inserted into the lumen of the blood vessel and the angioplasty balloon (6) is expanded in order to expand the area having the stenosis. If necessary, a stent (8) is placed to cover the afflicted area.

The problem in this treatment is that emboli can be formed during the course of the procedure which can rapidly reach the brain and cause injury and death. Emboli are especially prone to be formed when instruments pass into or through the stenosis and/or the angioplasty balloon is expanded or while the stent is placed and expanded.

In reference to the prior art, US 5,011,488 (Ginsburg) discloses a vascular catheter system in which a flexible tube member (18) with a radially extending end portion (16) is extended from within an outer tube (12). The flexible member (18) may be extended from within the tube (12) to a position beyond a clot or thrombus located within a blood vessel (BV), the flexible end portion (16) being radially extended to conform to the interior wall of the vessel (BV). The radially extended flexible member (16) is subsequently withdrawn dislodging and drawing the clot material towards the outer tube (12). Suction, or aspiration, is applied via a sealing box (44) to assist in removal of the clot material from the vein and through the catheter. An inner tube (14) located within the outer tube (12) also comprises an extendable, generally conical, portion (30) that is used to collect the clot material as the material; is drawn back by the flexible member (18).

Methods are known in the art to monitor the flow of blood, and thus indicate the existence of emboli. One such system, known as the transcranial Doppler monitoring system, measures the direction and velocity of blood flow in an artery in the brain, such as in the middle cerebral artery. Surgery and endovascular procedures on the carotid artery may be carried out while the transcranial Doppler system measures the speed of the blood flow in an artery.

In normal conditions, the blood stream has an anterograde flow, i.e. it goes forward, so that in the carotid artery it goes from the common carotid artery to the external and internal branches. In conventional surgery, first the artery is occluded and the blood flow is interrupted distal to the occlusion, before treating the stenosis. However, this surgery has some risk, since occlusion of the blood flow can cause cerebral ischemia. Therefore, in conventional surgery after the vessel has been occluded some surgeons use a shunt which is placed to allow for the temporary conduct of blood flow to the areas distal to the occlusion. Dissection of the artery and instrumentation to apply a shunt could generate the passage of emboli to the brain and consequent injury or even death of the patient. During carotid angioplasty and stenting particles are often produced by the procedure itself. Passing a guide wire through the stenosis, forcefully balloon dilating the stenosis, and,deploying and expanding stents can precipitate embolization to the brain.

Therefore, there is needed in the art for a device that allows for the removal of emboli during endovascular angioplasty.

The present invention recognizes that typically blood flow is not totally stopped distal to the surgical occlusion of a carotid vessel, because there exists anastomoses or intercommunications between arterial vessels by connecting cross branches, which allow blood to circulate distal to the occlusion. For instance, such collateral flow exists between the internal carotid artery and the branches of the external carotid artery. Additionally, the connections within the Circle of Willis (900) also allow for collateral blood flow. Previously, there has been use of retrograde perfusion on the venous side to provide blood flow to the brain. There has also been use of reversal of blood flow in the heart, such as using veins to bypass the arteries in the heart, or connecting the left ventricle of the heart to a vein to treat end stage cardiac disease.

The present invention allows blood flow to be reversed under control, thus emptying outside of the patient's body the emboli generated by the angioplasty, and utilizes the naturally occurring anastomoses, to allow for blood flow to areas distal to the occlusion during the relatively short periods of time that blood flow is reversed during the operative procedure.

The present invention recognizes that a funnel shaped balloon or occluder will direct all the blood flow and emboli inside the tube within the catheter to discharge outside of the patient's body where the blood may be cleansed of the emboli and then intravenously returned to the patient.

### Summary of the Invention

The invention comprises a tube placed inside a leading catheter and movable in an axial direction. The tube is provided with a distal end, a proximal end, a tubular body and an internal passageway. On the distal end of the tube there is an occluder, which is expandable against the vascular duct to occlude the anterograde blood flow. The occluder has a mouth for drainage of the retrograde blood flow from its distal end, through its internal passageway, through the tube to exit the patient at an outlet at the proximal end of the tube. The occluder preferably comprises a structure having a funnel shape with non-folding edges or may be in the form of a balloon in the shape of a funnel to direct all the flow and particles inside the tube. The tube at its proximal end may be provided with a plurality of branched connections. One of the branches connects to an outlet which is provided with a hemostatic valve and pump, which may be used to apply vacuum pressure, to establish retrograde blood flow and suction out emboli. Another of the branches connects to an inlet and provides an access way to its internal passageway. The tube may be provided with a plurality of branches each having an inlet. The branches are situated on the tube such that when the tube is inserted into the catheter the branches remain outside of the catheter.

In use, the distal end of the tube with the attached occluder is positioned into the proximal end of the catheter, extended through the catheter and then out of the distal end of the catheter. The occluder then expands against the vascular walls. When a balloon is used as the occluder, the expansion may be accomplished by means known in the art, such as the injection of saline solution or radio-opaque material into the balloon. When a non-balloon funnel is used as the occluder, the expansion may be accomplished by means known in the art, such as the passage of the occluder past the distal end of the catheter, where in the absence of tensile pressure from the walls of the catheter, the non-balloon funnel can spontaneously expand. Alternatively, when a non-balloon funnel is used as the occluder, the occluder may be placed within a sheath and the expansion may be accomplished by retracting the sheath in a proximal direction which allows the occluder to expand. Once the expansion is accomplished, the occluder has the shape of a goblet or funnel, and it occludes the anterograde flow of the blood stream in which it is placed and allows for the retrograde flow of blood through its mouth of drainage and then through the tube to exit the patient's body.

When the hemostatic valve is opened and vacuum suction is applied, a retrograde blood flow occurs that carries the emboli through the mouth of the occluder, and then through the tube to the exterior of the body to a collector, such as a bottle, or a cleansing system. The branch of the tube connected to the outlet may be provided with means known in the art for cleansing the blood of emboli. The means of cleansing the blood of emboli may include a filter known in the art, such as the Baxter filter or the Therumo filter. The hemostatic valve may be connected to a vacuum system known in the art, which comprises a pump for creating a negative pressure in the tube connected to the hemostatic valve. After the blood has been cleansed of emboli, the blood can be reintroduced to the patient intravenously. In this way, emboli are prevented from reaching the brain and removed from the blood collected outside the body before reinjecting the blood back to the patient.

A method against embolization, which does not form part of the invention, may comprise the use of the device in conjunction with vacuum suction, preferably at four stages of carotid angioplasty, 1) during guide wire advancement through the stenosis, 2) during balloon dilation, 3) during stent deployment, 4) during stent expansion, to allow for the reversal of blood flow to prevent emboli from reaching the brain and to allow the blood to be drained from the body, cleaned of emboli and reinfused into the patient's blood stream.

### BRIEF DESCRIPTION OF THE DRAWINGS

Figure 1 a is a longitudinal perspective view of the protective device with the occluder in its inoperative, contracted, position.
Figure 1b is a longitudinal perspective view of the protective device in which the occluder is shown beginning to exit from the distal end of the catheter and expanding against the blood vessel walls (not shown).

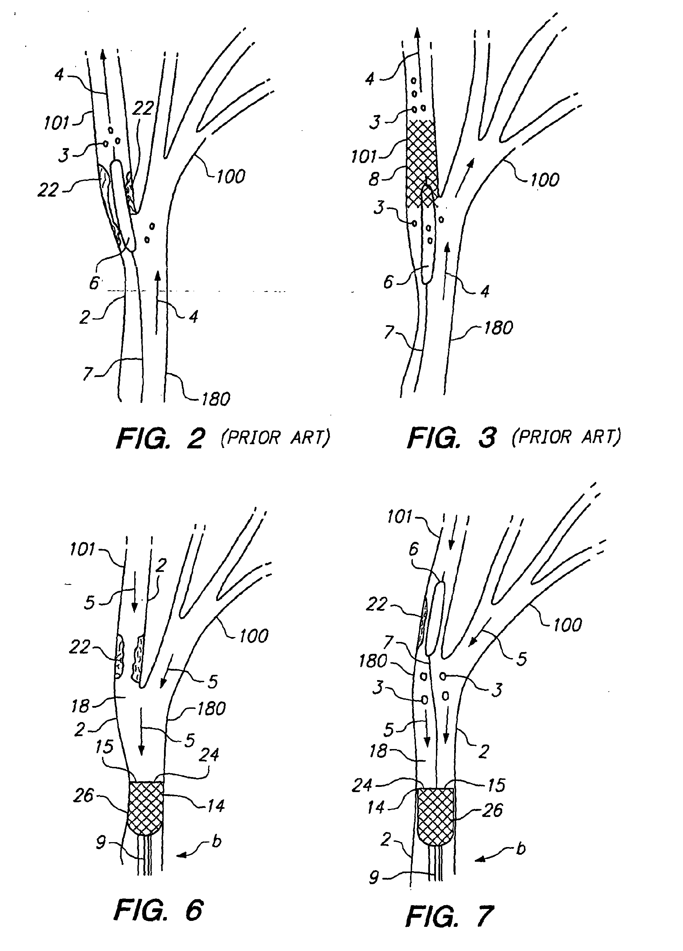

Figure 2 is a longitudinal view of the carotid artery, in which an angioplasty balloon expands the stenosis and causes the undesirable formation of emboli, as in the prior art, that then can advance distally to the brain, carried by the anterograde flow of the bloodstream.
Figure 3 is a longitudinal view of the carotid artery, in which the placement of an expander or stent as in the prior art, also generates dangerous emboli carried by the blood distally to the brain.
Figure 4 is a longitudinal partial view of the protective device of one embodiment of the present invention (the tube not being shown), being inserted into the carotid artery where the occluder begins to exit the leading catheter and to expand.
Figure 5 is a longitudinal partial view of the protective device of one embodiment of the present invention (the tube not being shown) in the carotid artery in which the expansion of the occluder against the vascular walls has been completed. It can be seen how the external edges of the occluder occlude the anterograde flow of blood, and prevent pooling or trapping of blood between the occluder and the blood vessel walls, while blood begins to flow retrograde through the mouth of drainage of the occluder and tube.
Figure 6 is a longitudinal view of one embodiment of the protective device in the carotid artery with the distal end of the occluder expanded in the shape of a funnel or goblet and where blood flow is reversed (as shown by the arrows) and is now retrograde when the hemostatic valve is open.
Figure 7 is a longitudinal view of one embodiment of the protective device in the carotid artery and an angioplasty balloon catheter in the internal carotid artery. It shows retrograde blood flow carrying emboli to the mouth of the drainage of the occluder.
Figure 8 is a longitudinal view of an embodiment of a portion of the protective device (catheter not shown), showing an occluder and the side port of the tube for draining blood.
Figure 9 is a schematic view of prior art showing a spherical balloon and arrows indicating where emboli may be caught on the balloon.
Figure 10A is a schematic view of the collateral circulation commonly found following occlusion of the internal carotid artery.
Figure 10B is a schematic view of the reduced collateral circulation following occlusion of the internal carotid artery, where there is anomalous insufficiency of segments of cerebral arterial circle of Willis.
Figure 11 is a schematic chart illustrating the step of one embodiment of a method, which does not form part of the invention, against embolization in carotid angioplasty.
Figure 12 is a longitudinal perspective partial view of an embodiment of the protective device of the present invention (catheter not shown) showing an embodiment wherein the occluder is within a retractable sheath.
Figure 13 is a longitudinal perspective partial view of an embodiment of the protective device of the present invention (catheter not shown) showing an embodiment wherein the occluder is expanded after the sheath has been retracted.
Figure 14 is a perspective partial view of a balloon occluder in its expanded position (with the catheter not shown).

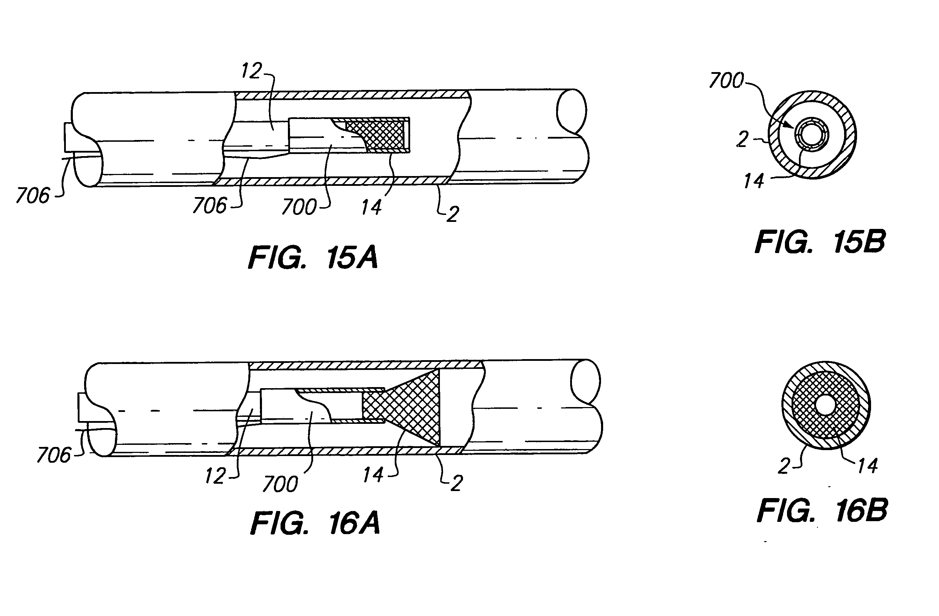

Figure 15A is one example of a longitudinal partial cross-section of the protective device (with catheter not shown) showing an embodiment of a non-balloon occluder in its contracted configuration.
Figure 15B is a horizontal cross-section of the protective device (with catheter not shown), as shown in Figure 15A, with the non-balloon occluder in its contracted configuration.
Figure 16A is one example of a longitudinal partial cross-section of the protective device (with catheter not shown) showing an embodiment of a non-balloon occluder in its expanded configuration.
Figure 16B is a horizontal cross-section of the protective device (with catheter not shown), as shown in Figure 16A, with the non-balloon occluder in its expanded configuration.

### Description of the Preferred Embodiments

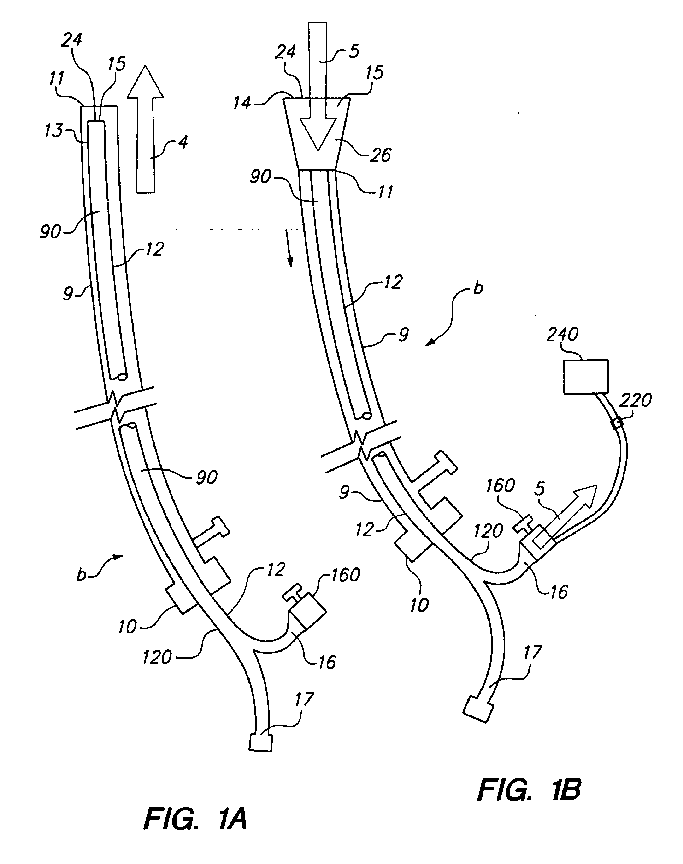

The protective device against embolization (b) in carotid angioplasty illustrated in FIG. 1b comprises a tube (12), inserted through a leading catheter (9), and an occluder (14) at the distal end (13) of the tube (12). The occluder may either be integral with the tube, or attached thereto. The tube (12) moves in an axial direction, i.e., longitudinally with respect to the leading catheter (9). The tube (12) comprises a cylinder having a distal end (13), a proximal end (120) and a body inside of which there is a cavity which forms an internal passageway (90).

The occluder (14) is inoperatively constrained inside the leading catheter (9) in a configuration in which the occluder has a distal rim (802) of reduced diameter. The occluder (14) is then passed out the distal end of the leading catheter (9) and is expanded against the vascular duct to occlude the anterograde blood flow (4).

The occluder (14) has a mouth (15) for drainage of emboli (3) into the internal passageway of the occluder. The occluder has a first configuration of reduced size and a second configuration of expanded size, the occluder being expandable from its first configuration to it second configuration. As with funnels in general, the occluder has a proximal rim (801) which forms a proximal first opening (800) within the occluder at its proximal end, which is narrower than the mouth (15) at its distal end when the occluder is in its expanded configuration. The occluder in its expanded configuration has a body in the shape of a funnel, the body comprising a funnel-shaped wall forming an internal passageway having a distal mouth and a proximal first opening (800). The first opening (800) forms a juncture with the opening of the tube (12) at the distal end (13) of the tube. Preferably, the first opening (800) of the occluder (14) is near the center of the body (26) of the occluder. Blood and emboli drain through the mouth (15) of the occluder (14), the first opening (800) of the occluder and pass through the distal end (13) of the tube (12) through its internal passageway (90) to the proximal end (120) of the tube (12) to exit the body of the patient through the outlet (16). The occluder (14) is expandable and is made of an expandable and impermeable material, as is known in the art. Materials with similar properties to known expanders can be used, such as the CORVITA^{™} stent, though this material should be combined with an impermeable material, since the occluder (14) must be able to function to occlude the passage of blood, as described herein. In one embodiment, the occluder is made of a shape memory metal (e.g. NITINOL™) which will transform in shape between its austensitic to martensitic states upon the application and removal of temperature (e.g., body heat) or stress. Alternatively; other elastic materials can be employed. As another example, a balloon occluder may be made from polyurethane braided with metal or from another biocompatible material.

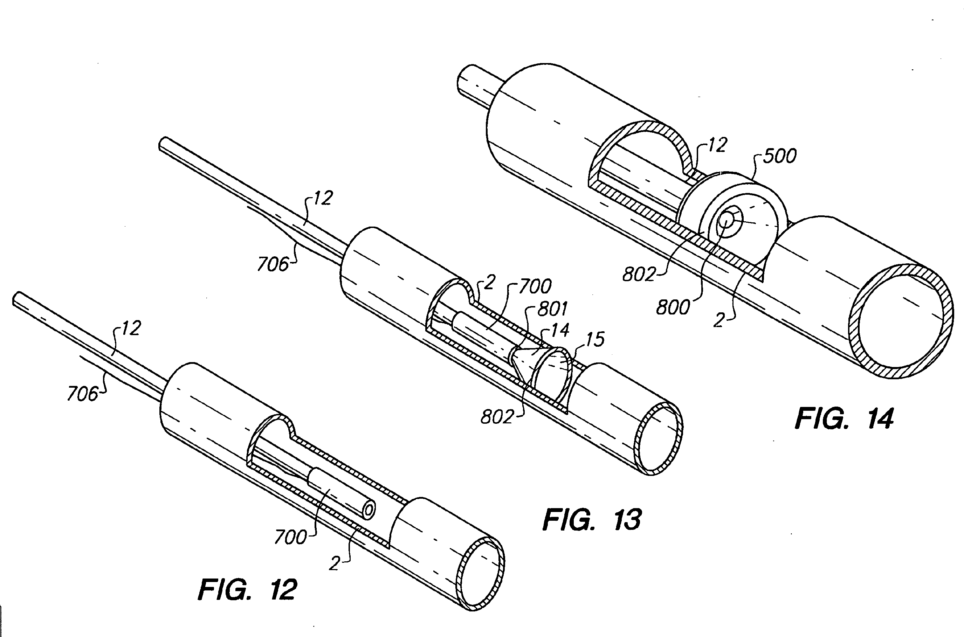

The occluder (14) preferably has a body (26) in the shape of a funnel. In one embodiment, the occluder may be as described above, but constructed from an inflatable balloon (500). Inflatable angioplasty balloons are known in the art. However, in accordance with the present invention, the balloon of the occluder has a funnel shape, as shown in Fig. 14. In an alternate embodiment, the occluder may be in the form of a non-balloon having a funnel shape with a central mouth of drainage (15) and a non-folding distal edge (24) which meets with the vascular duct walls (2) to prevent and occlude the anterograde passage of blood and direct the retrograde flow of blood and emboli through its mouth of drainage (15), as shown in Fig. 1 b.

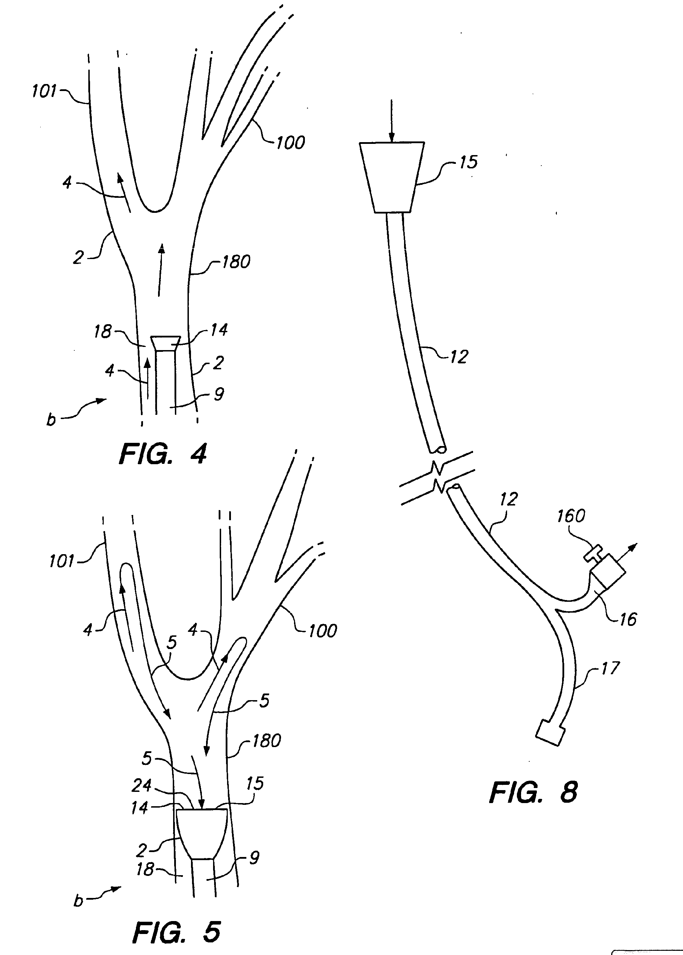

Furthermore, in the occluder's operative position, where it is projected to the exterior of the leading catheter, it undergoes a radial expansion by means of which it reaches a larger diameter than that of the leading catheter (9). Thus, in one embodiment the leading catheter (9) has a diameter of approximately 9 french or .30303 mm, the tube has a diameter of approximately 8 french or 2.72727 mm and the expanded occluder (14) is able to reach a diameter of 12 millimeters. In its expanded position against the vascular duct walls (2) the occluder (14) acquires a shape similar to that of a funnel or goblet, as shown in Fig. 5.

In an embodiment wherein the occluder (14) is a non-balloon funnel, the occluder (14) in its contracted position may be placed within a sheath (700). The sheath (700) is a cylinder having a cavity and is known in the art. The sheath may be composed of plastic, plastic combined with metal or any other suitable material (preferably biocompatible) as known in the art. Preferably, the sheath is dimensioned such that its width is slightly larger than the width of the occluder in its-contracted position such that the pressure of the interior walls of the sheath on the occluder holds the occluder (14) in its contracted position. In the preferred embodiment, the sheath is dimensioned such that its length is sufficiently long that, when the occluder is inserted into the sheath and the occluder is properly positioned in the patient at the level of intended use, the distal end of the sheath extends beyond the distal end of the occluder and the proximal end of the sheath extends outside of the patient's body. In an alternative embodiment, the sheath partially covers the occluder, the sheath sufficiently covering the occluder such that the occluder is restrained in its contracted, reduced diameter position. Thus, the retractable sheath can be retracted by the operator by pulling on the sheath portion outside the body, to pull the sheath back, exposing the occluder. In another embodiment, the sheath is attached to a means of extension, such as a wire, plastic filament, string or so forth (706) by adhesive, bonding, melting or other conventional means known in the art. The operator may manually pull on the device of extension to retract the sheath from the occluder and thus allow the occluder to expand. In such embodiment, the sheath may be dimensioned such that its length in combination with the length of the device of extension together forms a length whereby the means of extension extends outside of the patient's body and the occluder in its contracted position within the sheath is positioned in the patient at the level of intended use.

The proximal end (120) of the tube (12) may be provided with a plurality of branched connections (16)(17). One of the branches is an outlet (16) which is provided with a hemostatic valve (160) and vacuum pump mechanism (240). The vacuum pump mechanism is known in the art and, by way of example, the vacuum pump systems used in blood donations may be used here. One or more of the branches is an inlet (17) which provides an access way to the internal passageway (90) of the tube (12). The branches are situated on the tube (12) so that when the tube (12) is inserted into the leading catheter (9) the branches remain outside of the leading catheter (9). The tube may be made of material known in the art.

The leading catheter (9) is known in the art. The leading catheter (9) comprises a cylinder having a proximal end (10) and a distal end (11) and has a flexible body inside of which there is a cavity. Interventional elements, for example, such as a guide wire (7), an angioplasty balloon (6) and a stent (8) may be passed through the leading catheter (9) and may be passed through the tube (12) within the leading catheter (9).

The protective device against embolization (b) is used in carotid angioplasty or any other suitable procedure to form a system that allows for the drainage of emboli (3) from a patient undergoing carotid angioplasty. Angioplasty is a surgical procedure to reduce or remove the stenosis (22) of a vascular duct wall (2).

The protective device against embolization (b) operates as follows. A leading catheter (9) is introduced into the lumen (18) of an arterial blood vessel having a blood stream which normally has an anterograde flow (4). Typically, in carotid angioplasty the leading catheter is introduced through the femoral artery of the patient. The occluder (14), as connected to the tube (12), is inserted into the proximal end (10) of the leading catheter (9) and the occluder (14) is at this time inoperative in the interior of the leading catheter (9), that is, constrained in the leading catheter (9) in a configuration of reduced size. By exerting pressure on the tube (12) the occluder (14) is passed distally through the cavity of the leading catheter (9) and exits the leading catheter (9) at its distal end (11). The occluder (14) then expands or is then expanded against the vascular duct walls (2).

If a balloon occluder is displayed, the balloon can be expanded by the introduction of saline solution, radio-opaque material or as otherwise known in the art.

A non-balloon occluder having a funnel shape may expand naturally when it passes the distal end of the leading catheter and is thereby released from the tensile pressure of the walls of the leading catheter. Likewise, a shape memory metal occluder can expand by virtue of the application of temperature to the occluder (i.e., the warming due to the exposure to body heat) and/or the removal of stress (i.e., the passage of the occluder past the restraining tubular walls). Thus, the non-balloon occluder is moved from its position of constraint within the leading catheter (9) to a position of exterior projection past the distal end (11) of the leading catheter (9).

In an alternative embodiment, a non-balloon occluder (14) in its contracted position, as connected to the tube (12), may be placed within a sheath (700) before the occluder (14) and tube (12) are introduced into the patient through the catheter (9). In this embodiment the non-balloon occluder (14) within the sheath (700) and tube (12) may then be inserted into the proximal end (10) of the leading catheter (9) and the occluder may be passed distally through the cavity of the leading catheter and exit the leading catheter at its distal end, and yet the sheath (700) will continue to constrain the occluder (14) in the occluder's contracted position. The sheath may be of such length that it extends outside of the patient's body or the sheath may be attached to a device of extension which extends outside of the patient's body. Respectively, either the end of the sheath extending outside of the patient's body or the end of the device of extension which extends outside of the patient's body may be pulled, thus retracting the sheath from around the occluder and allowing the occluder to expand against the vascular duct walls (2).

Once the expansion is complete (preferably to the shape of a funnel or goblet) the distal non-folding edge of the non-balloon occluder or the inflated balloon occluder will press against the vessel wall, blocking anterograde flow of the blood stream. The funnel shape further eliminates the creation of pockets where emboli can collect, which is a problem in the prior art.

After expansion of the occluder, endovascular procedures, such as carotid angioplasty, may be performed. For example, a guide wire, balloon of angioplasty, a stent or any other suitable apparatus or instrument may be passed through the cavity within the tube and mouth of the occluder to reach the area of the vascular walls affected by the stenosis for appropriate treatment.

After expansion of the occluder and at the appropriate times when the operator desires to drain the emboli the surgeon may open the hemostatic valve (160) and a retrograde flow (5) occurs, which carries the emboli (3) generated by the operation through the mouth of the occluder (15), through the internal passageway (90) of the tube (12) and through the outlet (16) to exit the body of the patient. This retrograde flow is directly opposite the normal direction of blood flow and directs the blood away from the brain. After the blood and emboli exit the body of the patient, the blood may be cleansed of emboli (e.g., using a blood filtration device, as known in the art) and the blood may be intravenously reintroduced into the patient. Thus, the protective device against embolization prevents emboli (3) from reaching the brain.

The occluder may be returned to its contracted position when the expanded configuration of the occluder is not needed to drain blood and emboli. In one embodiment of a non-balloon occluder, the tube attached to the occluder may be moved proximally until the occluder is returned to its contracted configuration within the catheter. In another embodiment of a protective device against embolization comprising a non-balloon occluder connected to a tube, the sheath (700) may be pushed distally so that the occluder is returned to its contracted configuration within the sheath. A balloon occluder may be returned to its contracted position by the removal of the saline solution, radio-opaque material or as otherwise known in the art.

In particular, a new method against embolization in the carotid angioplasty, which does not form part of the invention, may make use of the protective device, as described below.

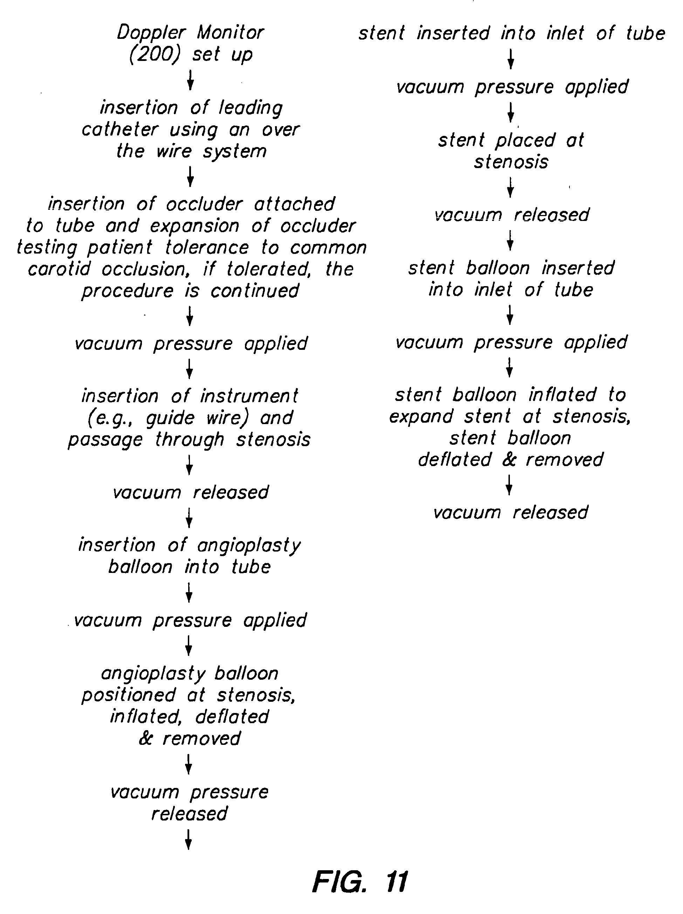

The protective device against embolization (b) may be used in angioplasty, and particularly in carotid angioplasty according to the following method of procedure, which is illustrated in schematic form in Fig. 11 and do not form part of the invention. At the start of the procedure a Doppler monitor (200) or transducer is preferably applied according to conventional means to monitor blood flow distal to the occlusion. For example, in carotid angioplasty a transcranial Doppler monitor is positioned over the temporal bone or a transducer is applied to the neck according to conventional methods for monitoring the blood flow in the patient of the common carotid artery (180), the external carotid artery (100), internal carotid artery (101) or the middle cerebral artery. This transcranial Doppler monitor will allow the physician to monitor the direction of blood flow in subsequent steps.

After the Doppler monitor (200) is set up, a leading catheter (9) is inserted into the patient and passed into the appropriate artery, which in the case of carotid angioplasty, is preferably the common carotid artery (180). The catheter is preferably not passed into or through the stenosis, to minimize the formation of potentially dangerous emboli. Next, the occluder (14) attached to the tube (12) is passed through the leading catheter and positioned proximal to the stenosis also preferably without passing into or through the stenosis. The occluder (14) is then expanded until the distal rim or edges of the occluder are flush with the blood vessel walls. Most of the patients will tolerate this common carotid temporary occlusion, some few will not, in this latter group of patients this procedure should be abandoned. The operator then applies vacuum suction to drain the emboli and determines when there is reversal of blood flow distal to the occlusion (e.g., using the transcranial Doppler monitor, or some other suitable device or method, such as a transducer or so forth). When reversal of flow is achieved, then the surgeon may pass an instrument, such as, a guide wire and an angioplasty balloon catheter through the tube (12) and pass it through the mouth (15) of the occluder (14) past the stenosis. Because reversal of flow has been established prior to the passing of any instruments past the stenosis which can dislodge emboli, the emboli will flow in a retrograde direction into the mouth of the occluder and flow through the protective device to exit the body of the patient. The vacuum pressure is released after the instrument has been passed past the stenosis and the emboli have been drained. In this manner, the prior art risk of creating emboli which became directed to the brain is reduced or eliminated.

More particularly, in carotid angioplasty the procedure may be categorized in four stages and in each of the four stages negative pressure may be applied at the appropriate times through the use of the protective device against embolization (b) to drain emboli.

In a first stage, a Doppler monitor (200) is set up. Then, a leading catheter (9) is inserted into the patient and passed into the common carotid artery (180) using a guide wire system (over the wire system) taking care not to traverse the stenosis with the end of the guide wire, typically the end of the guide wire should be positioned in the external carotid artery (without insertion into or through a stenosis). Next, the tube (12) with the attached occluder (14) is passed through the leading catheter and the occluder is positioned proximal to the stenosis. The occluder (14) is then expanded. In stage 1, prior to passing an instrument, such as a guide wire (7) through the stenosis, vacuum suction is applied by a vacuum pump mechanism (240) attached to the outlet (16). Such vacuum suction is applied until the flow is reversed (5) in the brain distal to the stenosis and is continued until the maneuver of passing the instrument, such as a guide wire, through the stenosis is completed. Typically, at this stage 1 the vacuum suction is applied for approximately 10 seconds. The vacuum suction will drain any emboli which may have been dislodged by the passage of instrumentation through the stenosis. Use of the Doppler monitor (or other suitable monitoring device, e.g., a transducer) will indicate when reverse flow is achieved in the brain distal to the stenosis.

In the second stage, an angioplasty catheter with an angioplasty balloon may then be introduced into the tube (12) within the leading catheter (9) through the inlet (17). In stage 2, in the pre-dilation state, the angioplasty catheter with angioplasty balloon is positioned at the level of the stenosis. In stage 2, at the time that the angioplasty catheter with angioplasty balloon is positioned near the-level of the stenosis and prior to dilation of the angioplasty balloon, the vacuum is again applied until the flow of blood is reversed distal to the stenosis by the pump mechanism attached to the outlet (16) and is continued until the angioplasty balloon is properly positioned at the level of the stenosis, the angioplasty balloon is inflated by introduction of saline solution, radio-opaque material, or as otherwise practiced in the art, and the angioplasty balloon is removed proximally to the stenosis. Typically, at this stage the vacuum suction is applied for approximately 15-20 seconds. The inflation of the angioplasty balloon breaks apart the stenosis and allows emboli to be released into the blood stream. The vacuum suction will then drain any emboli which may have been dislodged. Use of the Doppler monitor or suitable device will indicate when reverse flow is achieved in the brain distal to the stenosis. After the angioplasty balloon is deflated, the angioplasty balloon is withdrawn from the patient's body.

In stage 3, a stent is introduced through an inlet (17) into the tube (12) within the leading catheter (9). At the time the stent nears the stenosis the vacuum suction is applied until the flow is reversed distal to the stenosis and the stent is located at the stenosis. Typically, at this stage vacuum pressure is applied for approximately 30 seconds. The vacuum will drain any emboli which may have been dislodged. Use of the Doppler monitor or the like will indicate when reverse flow is achieved in the brain distal to the stenosis.

In stage 4 a stent balloon, also known as an inflation balloon (i.e. a balloon used to inflate a stent), is inserted into the inlet (17) into the tube (12) within the leading catheter (9). At the time the stent balloon nears the stenosis the vacuum suction is applied until the flow is reversed distal to the stenosis and the vacuum pressure continues to be applied until the stent balloon is inflated to expand the stent at the stenosis, the stent balloon is deflated and is removed proximally to the stenosis. Typically, the vacuum suction is applied for approximately 15-20 seconds at this stage. The vacuum will drain any emboli which- may have been dislodged. Use of the Doppler monitor or the like will indicate when reverse flow is achieved in the brain distal to the stenosis.

Preferably the reverse flow of blood is established only intermittently during the procedure, for brief periods. Although it is possible to sustain continuous reverse flow throughout the procedure, such sustained reverse flow is not preferred due to the undesirability of depriving the brain of oxygen for extended periods. In the preferred embodiment, during each stage of a procedure which could result in the dislodging of emboli, reverse flow is initiated only so long as needed to ensure that all potential emboli are fully drained and removed from the bloodstream. Accordingly, the vacuum suction periods provided are representative of periods believed useful in practice, but slightly shorter or longer periods may be necessary or useful in a particular procedure or type of procedure or for a particular patient.

Use of this method, which does not form part of the invention, typically results in the drainage of approximately 300 cc of blood, but the amount of blood drained may vary. The blood which is drained may be passed through a filter system (220), and be cleansed outside of the body of the patient by means well known in the art. After the blood has been cleansed, the blood may be reintroduced intravenously into the patient.

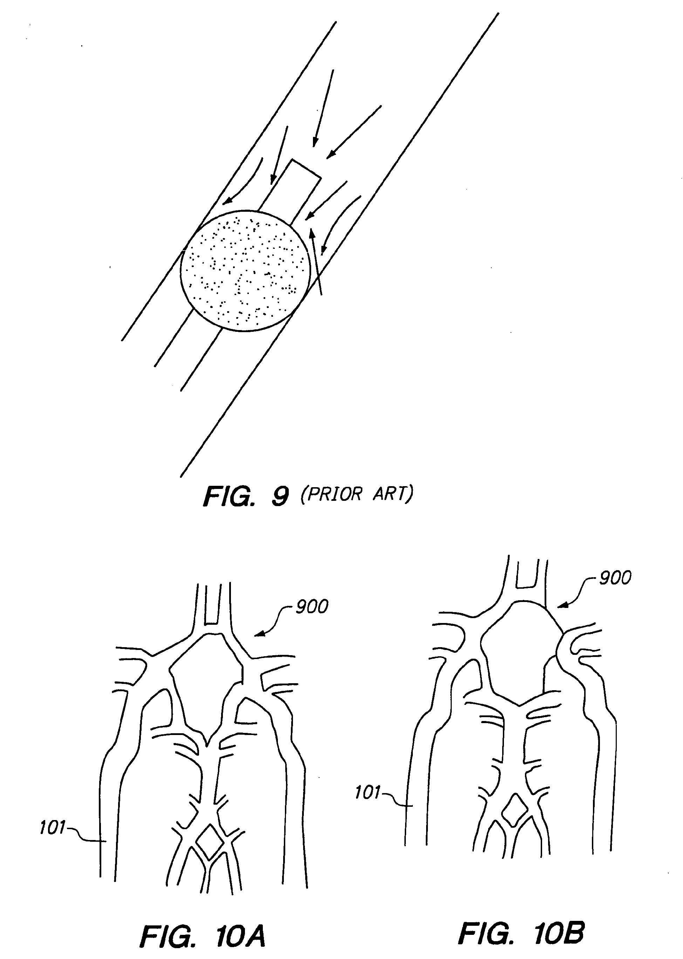

Thus, as described above, the method, which does not form part of the invention, against embolization reverses the blood of flow in the brain distal to the stenosis at four stages during the carotid angioplasty procedure. Alternatively the method, also not in accordance with the invention, against embolization may comprise anyone of the four stages or any combination of the four stages. The total period of reversal of blood flow in the brain distal to the stenosis is approximately one minute. In a typical patient, blood flow may be reversed for up to approximately three minutes before there is likely to be any danger from ischemic. FIG. 10A illustrates a schematic drawing of circulation, as it typically occurs in the segments of the cerebral arterial Circle of Willis.

Less than five percent of patients, however, are likely to have an anomalous insufficiency of segments of the cerebral arterial Circle of Willis. Anomalous insufficiency of segments of cerebral arterial Circle of Willis is known to reduce collateral flow and may result in ischemic where blood flow is reversed for even a relatively short time of three minutes or less. FIG. 10 B illustrates a schematic drawing of anomalous insufficiency of segments of the cerebral arterial Circle of Willis.

It is significant to recognize that suction or vacuuming can also be applied without achieving the purpose of reversing the flow in the internal carotid artery distal to the stenosis. Flow reversion is related to the amount of blood drained per unit of time and time of suction. It is therefore important to use the Doppler monitor (200) or the like to ascertain that reversal of blood has occurred when the surgeon is attempting to remove the emboli by applying suction.

This protective device (b) is applicable in angioplasties aimed to the treatment of arterial stenosis (22) that affects vascular duct walls (2). In particular, the protective device (b) is applicable in carotid angioplasties where there is a stenosis (22) in the carotid artery. The carotid artery is composed of the common carotid artery (180) and its derivations in the external carotid artery (100) and internal carotid artery (101). Inside the vascular duct, the blood stream normally has an anterograde flow (4), i.e. from the common carotid artery (1) to the branches (100) and (101).

In alternative -embodiments of the invention, the apparatus can be used in other procedures and repairs of the anatomy, where there is a risk of forming dangerous emboli.

Having described this invention with respect to its preferred embodiments, it is to be understood that the description is not meant as a limitation since further variations or modifications may be apparent or may suggest themselves to those skilled in the art. It is intended that the present application cover such variations and modifications as fall within the scope of the appended claims.

## Claims

1. A device (b) for removing emboli generated during carotid angioplasty comprising:
a guide catheter (9) having proximal and distal ends (10, 11), and a lumen extending therebetween;
a tubular member (12) slidably disposed within the lumen of the guide catheter (9) and an occluder (14) at a distal end (13) of the tubular member (12), the tubular member (12) having two proximal branches, the branches including an outlet
port and an access port (16, 17), the distal end (13) having an opening communicating with a drainage port (15) of the occluder (14), and a lumen (90) extending between the outlet and access ports (16, 17) and the distal opening, the tubular member (12) having a retracted position wherein the occluder (14) is disposed within the guide catheter (9) and has a retracted
diameter suitable for endoluminal insertion, and an extended position, wherein the occluder (14) extends beyond the distal end (11) of the guide catheter (9) and has an expanded diameter adapted to occlude anterograde blood flow in a vessel; and
a hemostatic valve (160) disposed on the outlet port (16) for controlling the flow of blood therethrough in a reverse retrograde direction and suction out emboli generated during an angioplasty procedure.

2. The device (b) of claim 1, wherein the occluder (14) further comprises a non-folding edge.

3. The device (b) of claim 1 or 2, wherein the occluder (14) is self-expanding and further comprises a fluid impermeable material.

4. The device (b) of claim 1, 2 or 3, wherein the occluder (14) assumes a goblet shape when the tubular member (12) is moved to the extended position.

5. The device (b) of claim 1, 2, 3 or 4, wherein the guide catheter (9) has a diameter of 9 French.

6. The device (b) of any one of claims 1 to 5, wherein the occluder (14) has a diameter of 12 millimeters when the tubular member (12) is in the extended position.

7. The device (b) of any one of claims 1 to 6, wherein the access port (17) is configured to allow interventional instruments to be advanced through the lumen (90) of the tubular member (12) and the drainage port (15) into the vessel.

## Patentansprüche

1. Vorrichtung (b) für das Beseitigen von Emboli, die während der Karotidangioplastie gebildet werden, die aufweist:
einen Führungskatheter (9) mit einem proximalen und einem distalen Ende (10, 11) und einem Hohlraum, der sich dazwischen erstreckt;
ein schlauchartiges Element (12), das verschiebbar innerhalb des Hohlraumes des Führungskatheters (9) angeordnet ist, und einen Occluder (14) an einem distalen Ende (13) des schlauchartigen Elementes (12), wobei das schlauchartige Element (12) zwei proximale Abzweigungen aufweist, wobei die Abzweigungen eine Austrittsöffnung und eine Zugangsöffnung (16, 17) umfassen, wobei das distale Ende (13) eine Öffnung aufweist, die mit einer Abflussöffnung (15) des Occluders (14) in Verbindung ist, und wobei sich ein Hohlraum (90) zwischen der Austrittsöffnung und der Zugangsöffnung (16, 17) und der distalen Öffnung erstreckt, wobei das schlauchartige Element (12) eine zurückgezogene Position, bei der der Occluder (14) innerhalb des Führungskatheters (9) angeordnet ist und einen eingezogenen Durchmesser aufweist, der für das endoluminale Einsetzen geeignet ist, und eine ausgezogene Position aufweist, bei der sich der Occluder (14) über das distale Ende (11) des Führungskatheters (9) hinaus erstreckt und einen expandierten Durchmesser aufweist, der ausgebildet ist, um einen anterograden Blutstrom in einem Gefäß zu verschließen; und
ein hämostatisches Ventil (160), das an der Austrittsöffnung (16) für das Steuern des Blutstromes dort hindurch in einer umgekehrten retrograden Richtung und das Heraussaugen der Emboli angeordnet ist, die während eines Angioplastieverfahrens gebildet werden.

2. Vorrichtung (b) nach Anspruch 1, bei der der Occluder (14) außerdem einen sich nicht faltenden Rand aufweist.

3. Vorrichtung (b) nach Anspruch 1 oder 2, bei der der Occluder (14) selbstexpandierend ist und außerdem ein fluidundurchlässiges Material aufweist.

4. Vorrichtung (b) nach Anspruch 1, 2 oder 3, bei der der Occluder (14) eine Kelchform annimmt, wenn das schlauchartige Element (12) in die ausgezogene Position bewegt wird.

5. Vorrichtung (b) nach Anspruch 1 , 2, 3 oder 4, bei der der Führungskatheter (9) einen Durchmesser von 9 Charr aufweist.

6. Vorrichtung (b) nach einem der Ansprüche 1 bis 5, bei der der Occluder (14) einen Durchmesser von 12 Millimeter aufweist, wenn sich das schlauchartige Element (12) in der ausgezogenen Position befindet.

7. Vorrichtung (b) nach einem der Ansprüche 1 bis 6, bei der die Zugangsöffnung (17) ausgebildet ist, damit die für einen Eingriff vorgesehenen Instrumente durch den Hohlraum (90) des schlauchartigen Elementes (12) und die Abflussöffnung (15) in das Gefäß transportiert werden können.

## Revendications

1. Dispositif (b) pour retirer des emboles formés au cours d'une angioplastie de la carotide, comprenant :
un cathéter de guidage (9), comportant des extrémités proximale et distale (10, 11) et une lumière s'étendant entre elles;
un élément tubulaire (12), agencé de manière coulissante dans la lumière du cathéter de guidage (9) et un dispositif d'occlusion (14) au niveau d'une extrémité distale (13) de l'élément tubulaire (12), l'élément tubulaire (12) comportant deux branches proximales, les branches englobant un orifice de sortie et un orifice d'accès (16, 17), l'extrémité distale (13) comportant une ouverture en communication avec le dispositif d'occlusion (14), et une lumière (90) s'étendant entre les orifices de sortie et d'accès (16, 17) et l'ouverture distale, l'élément tubulaire (12) ayant une position rétractée, dans laquelle le dispositif d'occlusion (14) est agencé dans le cathéter de guidage (9) et a un diamètre rétracté approprié pour une insertion endoluminale, et une position étendue, dans laquelle le dispositif d'occlusion (14) s'étend au-delà de l'extrémité distale (11) du cathéter de guidage (9) et a un diamètre étendu approprié pour bloquer l'écoulement de sang antérograde dans un vaisseau : et
une valve hémostatique (160), agencée sur l'orifice de sortie (16) pour contrôler l'écoulement du sang dans une direction rétrograde inverse et pour assurer le retrait par aspiration des emboles formés au cours d'une procédure d'angioplastie.

2. Dispositif (b) selon la revendication 1, dans lequel le dispositif d'occlusion (14) comprend en outre un bord non pliable.

3. Dispositif (b) selon les revendications 1 ou 2, dans lequel le dispositif d'occlusion (14) est auto-expansible et comprend en outre un matériau imperméable au fluide.

4. Dispositif (b) selon les revendications 1, 2 ou 3, dans lequel le dispositif d'occlusion (14) adopte une forme en gobelet lors du déplacement de l'élément tubulaire (12) vers la position étendue.

5. Dispositif (b) selon les revendications 1, 2, 3 ou 4, dans lequel le cathéter de guidage (9) a un diamètre de 9 French.

6. Dispositif (b) selon l'une quelconque des revendications 1 à 5, dans lequel le dispositif d'occlusion (14) a un diamètre de 12 millimètres lorsque l'élément tubulaire (12) se trouve dans la position étendue.

7. Dispositif (b) selon l'une quelconque des revendications 1 à 6, dans lequel l'orifice d'accès (17) est configuré de sorte à permettre l'avance d'instruments interventionnels à travers la lumière (90) de l'élément tubulaire (12) et l'orifice d'évacuation (15) dans le vaisseau.
